# FASCICULE DE BREVET EUROPEEN

(11) **EP 2 217 305 B1**
(45) Date de publication et mention de la délivrance du brevet: **19.06.2013**
(21) Numéro de dépôt: 08853886.3
(22) Date de dépôt: 12.11.2008
(51) Int. Cl.: A61M 5/14, A61M 39/24

(54) **DISPOSITIF D'ADMINISTRATION DE PLUSIEURS LIQUIDES DE TRAITEMENT MÉDICAL À UN PATIENT HUMAIN OU ANIMAL**
VORRICHTUNG ZUR VERABREICHUNG MEHRERER MEDIZINSICHER BEHANDLUNGSFLÜSSIGKEITEN AN MENSCHEN ODER TIERE
DEVICE FOR DELIVERING A PLURALITY OF MEDICAL TREATMENT LIQUIDS TO A HUMAN OR ANIMAL PATIENT

(30) Priorité: 12.11.2007 FR 0707912
(43) Date de publication de la demande: 18.08.2010
(73) Titulaire: Doran International, 69780 Toussieu (FR)
(72) Inventeur: BUISSON, Philippe, F-69780 Toussieu (FR)
(74) Mandataire: Maureau, Philippe
(86) Numéro de dépôt international: PCT/FR2008/052027
(87) Numéro de publication internationale: WO 2009/068806

(56) Documents cités:
- EP-A- 0 442 135
- WO-A-94/06489
- GB-A- 2 414 678
- US-A- 4 447 230
- US-A1- 2006 089 604

## Description

La présente invention concerne un dispositif d'administration de plusieurs liquides de traitement médical à un patient humain ou animal.

Il est connu, d'après le document WO 2004/045704, de réaliser un dispositif d'administration de plusieurs liquides de traitement médical à un patient, comportant un tube contenant plusieurs canaux sensiblement parallèles s'étendant sur toute la longueur du tube dont une extrémité, dite distale, est équipée de moyens de connexion à un élément formant cathéter, et dont l'autre extrémité, dite proximale, est connectée à un boîtier équipé de moyens de connexion de plusieurs récipients contenant chacun un liquide, le boîtier présentant des conduits de passage de liquide, chaque conduit de passage étant destiné à être connecté respectivement à un récipient et à un canal du tube.

En conditions d'utilisation, les moyens de connexion équipant l'extrémité distale du tube et l'élément formant cathéter délimitent une zone de convergence des différents liquides contenus dans les récipients connectés au boîtier.

Lorsque par exemple différentes perfusions par gravité sont reliées au boîtier de ce type de dispositif d'administration ou que des perfusions par gravité et des perfusions en pression positive contrôlées par exemple à l'aide de pousses seringues, sont reliées au boîtier de ce dispositif, une quantité de liquide peut, du fait des différences de pression régnant dans les différents récipients, s'écouler de la zone de convergence jusque dans un ou plusieurs récipients.

Il en résulte un mélange de différents liquides dans certains récipients et donc un doute sur la quantité de chaque liquide réellement administrée au patient, ce qui peut conduire à un mauvais traitement du patient.

De même, du fait des différences de pression entre la pression veineuse et les pressions régnant dans les différents récipients, du sang peut également s'écouler dans un ou plusieurs récipients et donc empêcher une administration satisfaisante des différents liquides.

En conséquence, le dispositif d'administration décrit dans le document WO 2004/045704 ne permet pas d'administrer de façon satisfaisante les liquides de traitement médical contenus dans les différents récipients connectés à ce dispositif.

Le problème technique à la base de l'invention consiste donc à fournir un dispositif d'administration de plusieurs liquides de traitement médical à un patient qui soit de structure simple, tout en assurant une administration satisfaisante des liquides contenus dans les différents récipients connectés au dispositif et un traitement satisfaisant du patient.

A cet effet, la présente invention concerne un dispositif d'administration selon la revendication 1.

La présence d'un clapet antiretour dans l'un des conduits de passage permet d'empêcher un écoulement de liquide de la zone de convergence des différents liquides contenus dans les récipients connectés au boîtier jusque dans le récipient correspondant, et donc d'assurer une administration satisfaisante du liquide contenu dans ce dernier.

En équipant chaque conduit de passage avec un clapet antiretour, il est alors possible d'empêcher un écoulement de liquide de la zone de convergence jusque dans chaque récipient, et donc d'assurer une administration satisfaisante des différents liquides de traitement.

En conséquence, lorsque la pression dans la zone de convergence est supérieure à celle de l'un des récipients du fait par exemple du raccordement de perfusions par gravité et de perfusions en pression positive (du type pousse-seringue) au boîtier du dispositif d'administration, le liquide contenu dans la zone de convergence ne peut pas s'écouler vers ce récipient grâce à la présence du clapet antiretour disposé dans le conduit de passage correspondant à ce récipient.

En outre, étant donné que chaque conduit de passage est agencé pour être connecté respectivement à un récipient et à un canal du tube, les liquides contenus dans les différents récipients connectés au dispositif d'administration selon l'invention peuvent être administrés au patient sans mélange préalable au niveau du dispositif d'administration.

Ces dispositions permettent de contrôler avec précision l'administration de chaque liquide contenu dans les différents récipients connectés au dispositif d'administration, et plus particulièrement la quantité administrée et le débit d'administration de chaque produit de traitement médical.

Un tel contrôle de la quantité de produits administrée au patient et du débit d'administration de ces produits peut s'avérer primordial pour le traitement d'un patient.

Par exemple, dans le cas de l'insulinothérapie, il a été constaté qu'une administration d'insuline et d'antibiotique à un patient en utilisant une voie commune d'injection ne permet pas de contrôler avec précision la quantité de produits administrée au patient, et de ce fait augmente de manière importante les risques d'hypoglycémie. A l'inverse, une administration d'insuline et d'antibiotique à un patient en utilisant un dispositif d'administration selon l'invention permet d'éviter de tels risques d'hypoglycémie du fait que l'insuline et l'antibiotique sont administrés sans mélange préalable au niveau du dispositif.

L'administration d'un vasopresseur à un patient doit être effectuée sensiblement à débit constant afin d'éviter une soudaine augmentation ou chute de la pression artérielle du patient, et nécessite donc un contrôle précis du débit d'administration du vasopresseur.

L'utilisation du dispositif d'administration selon l'invention assure une administration directe du vasopresseur au patient sans mélange préalable dans le dispositif avec un autre produit, ce qui permet de contrôler avec précision son débit d'administration et donc d'éviter les risques de variation soudaine de la pression artérielle du patient.

Avantageusement, les conduits de passage du boîtier convergent vers le centre du boîtier dans une zone de diamètre correspondant sensiblement à celle du tube, chaque conduit possédant un orifice de sortie orienté perpendiculairement au plan dans lequel sont disposés les conduits, chaque orifice étant en regard d'un canal du tube.

De préférence, chaque orifice de sortie est prolongé par une broche creuse engagée dans le canal correspondant du tube.

Selon un mode de réalisation de l'invention, le boîtier est de forme circulaire, et le tube est connecté au boîtier de manière axiale.

De préférence, le tube est évasé au niveau de son extrémité proximale.

Selon encore un autre mode de réalisation de l'invention, le boîtier comprend un manchon cylindrique agencé pour recevoir par emboîtement à force l'extrémité proximale du tube.

Avantageusement, chaque conduit du boîtier est fermé au niveau de la paroi extérieure du boîtier par un obturateur perforable, fendu ou à clapet mobile.

De préférence, chaque conduit du boîtier délimite, au niveau de son extrémité tournée vers l'extérieur, un siège dans lequel est monté un embout tubulaire de type luer formant un moyen de connexion du récipient correspondant et servant au logement de l'obturateur correspondant.

Selon une autre caractéristique de l'invention, chaque conduit délimite une chambre dans laquelle est monté mobile en translation un piston formant clapet antiretour, le piston étant mobile entre une première position permettant le passage de liquide du récipient correspondant vers le canal correspondant du tube et une seconde position empêchant le passage de liquide du canal correspondant vers le récipient correspondant.

Avantageusement, chaque obturateur est tubulaire et présente une première extrémité fermée et une seconde extrémité ouverte, et chaque piston est agencé pour obturer l'extrémité ouverte de l'obturateur correspondant lorsqu'il est dans sa seconde position.

De préférence, chaque piston présente une extrémité amincie agencée pour obturer l'extrémité ouverte de l'obturateur correspondant lorsque le piston est dans sa seconde position.

Avantageusement, l'extrémité amincie de chaque piston est tronconique.

Selon un mode de réalisation de l'invention, chaque piston présente un diamètre correspondant au diamètre de la chambre correspondante, et en ce que chaque piston comprend au moins une rainure longitudinale ménagée sur sa surface extérieure et agencée pour permettre le passage de liquide du récipient correspondant vers le canal correspondant du tube.

De préférence, le dispositif d'administration comporte une embase sensiblement cylindrique sur laquelle est monté le boîtier, l'embase formant un tambour d'enroulement pour le tube.

Avantageusement, le dispositif d'administration comporte des capteurs montés sur la face extérieure du boîtier, chaque capteur étant agencé pour mesurer au moins une valeur caractéristique de l'écoulement de liquide dans un conduit de passage.

De toute façon, l'invention sera bien comprise à l'aide de la description qui suit, en référence au dessin schématique annexé représentant, à titre d'exemple non limitatif, une forme de réalisation préférée du dispositif d'administration selon l'invention.
Figure 1 en est une vue en perspective.
Figure 2 en est une vue en perspective dans laquelle le flasque supérieur du boîtier a été enlevé.
Figure 3 en est une vue éclatée en perspective.

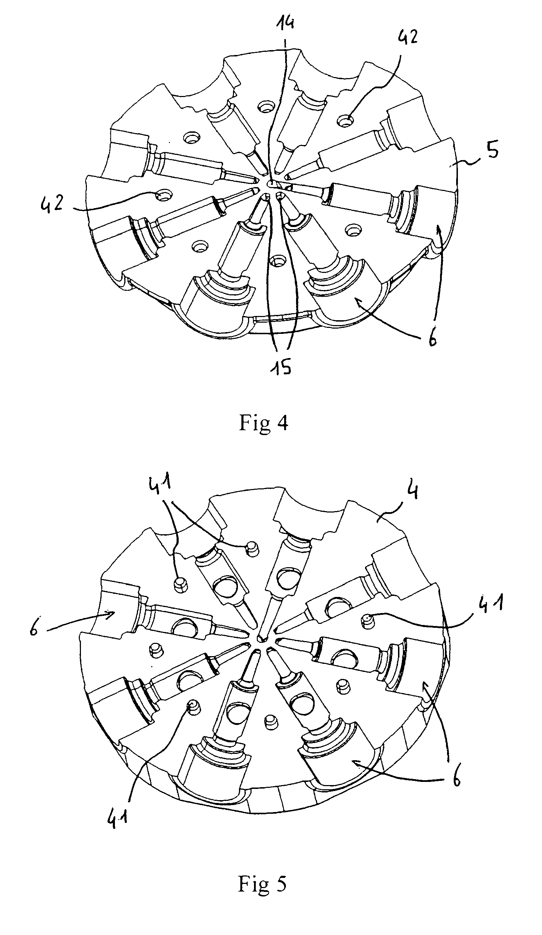

Figure 4 est une vue en perspective de dessus du flasque inférieur du boîtier.
Figure 5 est une vue en perspective de dessous du flasque supérieur du boîtier.

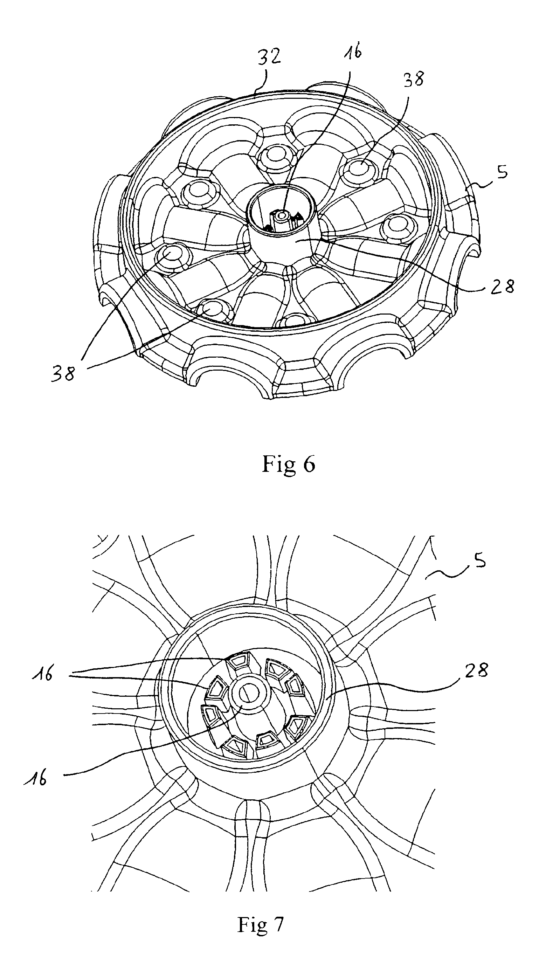

Figure 6 est une vue en perspective de dessous du flasque inférieur du boîtier.
Figure 7 est une vue à l'échelle agrandie du élément de figure 6.
Figures 8 et 9 sont des vues partielles en coupe du boîtier montrant un piston d'un conduit de passage dans deux positions de fonctionnement.

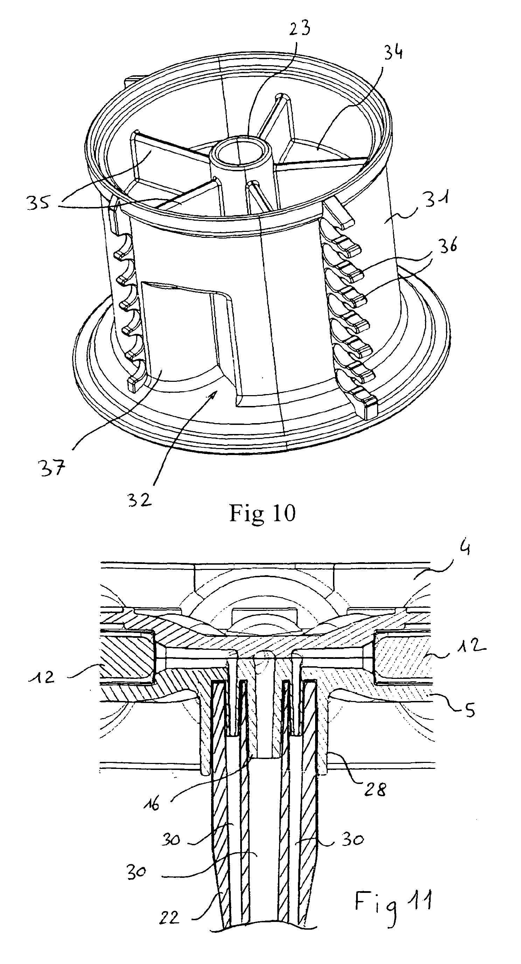

Figure 10 est une vue en perspective de dessus de l'embase.
Figure 11 est une vue partielle en coupe du boîtier montrant la connexion entre le boîtier et le tube.

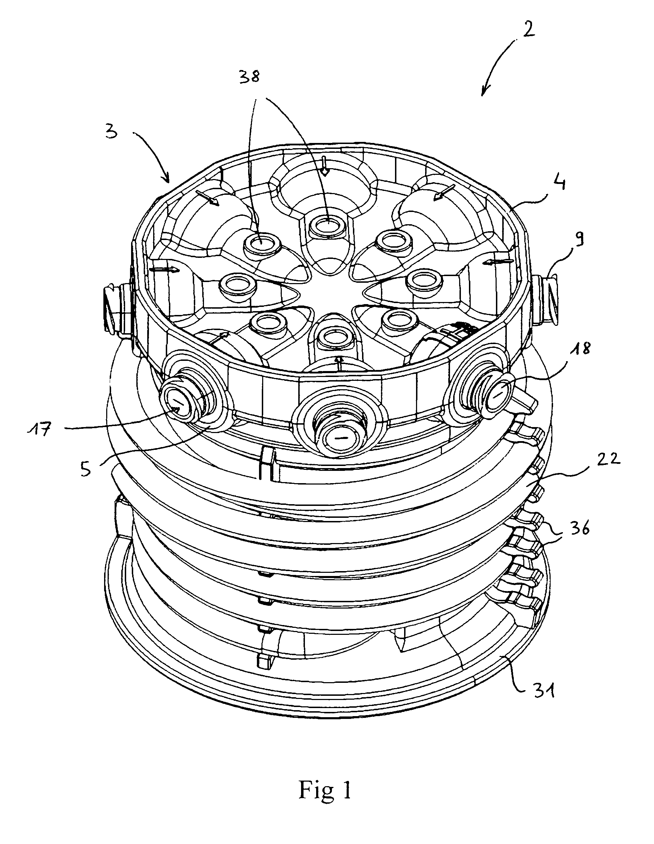

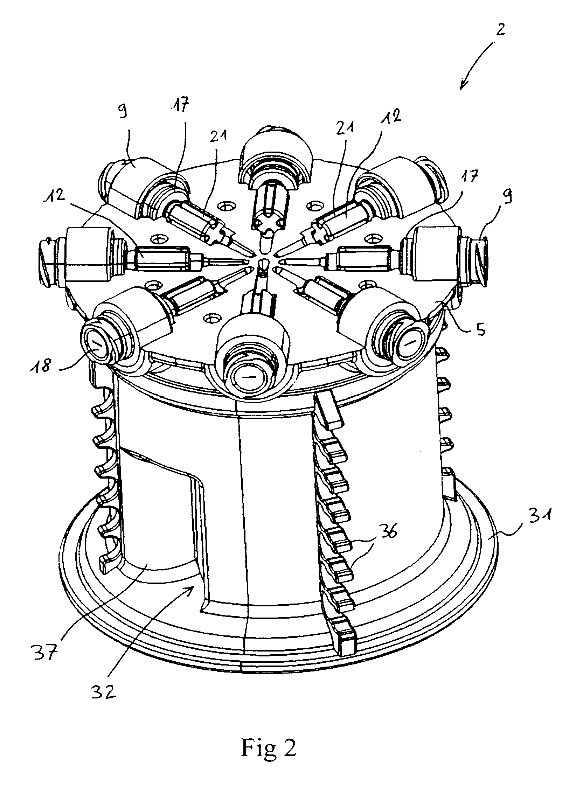

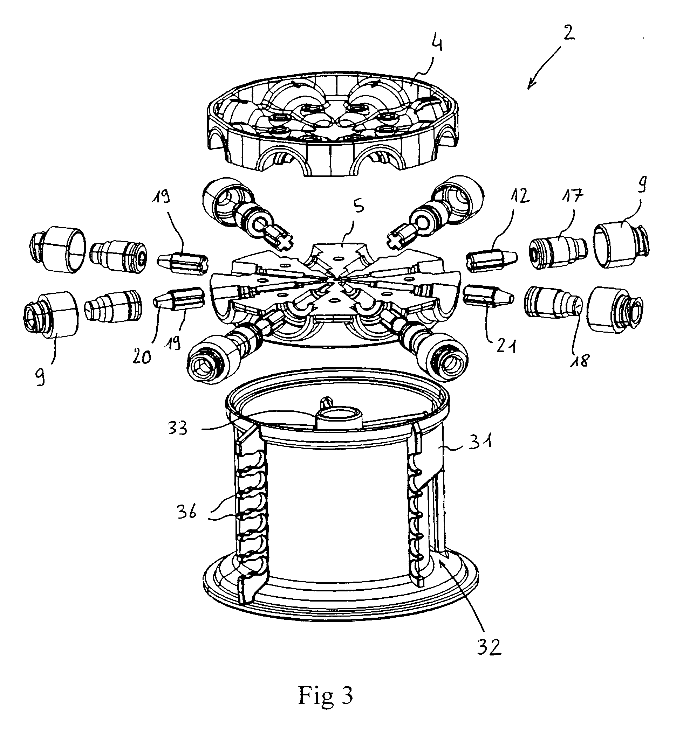

Les figures 1 à 3 représentent un dispositif d'administration 2 de plusieurs liquides de traitement médical à un patient humain ou animal.

Le dispositif d'administration 2 comporte un boîtier 3 constitué par une flasque supérieur circulaire 4 et un flasque inférieur circulaire 5. Les deux flasques 4, 5 comportent chacun des empreintes 6 complémentaires pour former des conduits de passage de liquide 7 s'étendant radialement de l'extérieur du boîtier 3 vers le centre du boîtier. Les conduits de passage 7 sont régulièrement espacés.

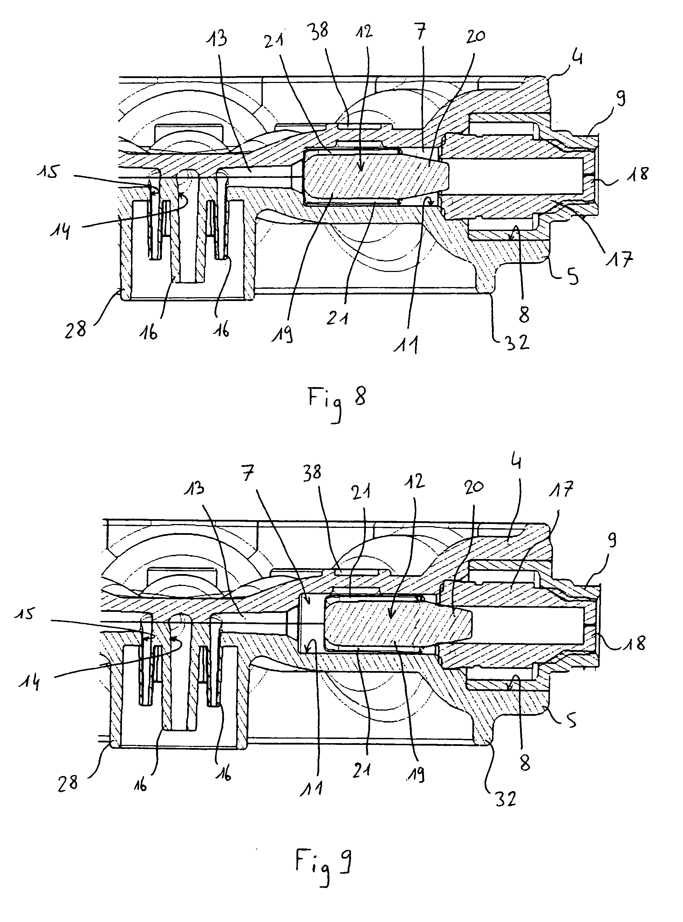

Comme montré plus particulièrement sur les figures 8 et 9, chaque conduit de passage 7 délimite, de l'extérieur vers l'intérieur, un siège cylindrique 8 dans lequel est monté un embout tubulaire 9 de type luer destiné à être connecté à un récipient contenant un liquide, une chambre cylindrique 11 dans laquelle est monté mobile en translation un piston 12 formant clapet antiretour, et un canal de passage 13 convergeant vers le centre du boîtier 3.

Le flasque inférieur 5 comporte des orifices de sortie 14, 15 parallèles orientés perpendiculairement au plan dans lequel sont disposés les conduits de passage 7. Le flasque inférieur 5 comporte un orifice de sortie central 14 et des orifices de sortie périphériques 15.

Le canal de passage 13 de chaque conduit de passage 7 est prolongé par un orifice de sortie périphérique 15 ménagé dans le flasque 5 tandis que l'un des canaux de passage 13 est également prolongé par l'orifice de sortie central 14 de sorte que le conduit de passage 7 correspondant comporte deux orifices de sortie.

Chaque orifice de sortie 14, 15 est prolongé par une broche creuse 16 solidaire du flasque inférieur 5 et orientée perpendiculairement au plan dans lequel sont disposés les conduits de passage 7.

Chaque conduit de passage 7 est fermé au niveau de la paroi extérieure du boîtier 3 par un obturateur 17 logé dans l'embout correspondant 9. Chaque obturateur 17 est tubulaire et présente une première extrémité tournée vers l'extérieur fermée par une membrane fendue 18 et une seconde extrémité ouverte tournée vers l'intérieur.

Chaque piston 12 comporte une première portion 19 sensiblement cylindrique présentant un diamètre correspondant à celui de la chambre correspondante 11, et une seconde portion tronconique 20 tournée vers l'obturateur correspondant 16.

Chaque piston 12 comprend des rainures longitudinales 21 ménagées sur la surface extérieure de sa première portion 19 et agencée pour permettre un passage de liquide provenant du récipient correspondant vers le canal de passage correspondant 13.

Chaque piston 12 est mobile entre une première position dans laquelle la portion tronconique 20 du piston est situé à distance de l'extrémité ouverte de l'obturateur correspondant 16 et permet le passage de liquide du récipient correspondant vers le canal de passage correspondant 13 et une seconde position dans laquelle la portion tronconique 20 du piston obture l'extrémité ouverte de l'obturateur correspondant 17 et empêche le passage de liquide du canal de passage correspondant 13 vers le récipient correspondant.

Le dispositif d'administration 2 comporte également un tube flexible 22 contenant plusieurs canaux 30 sensiblement parallèles s'étendant sur toute la longueur du tube. L'extrémité proximale du tube est connectée au boîtier 3 tandis que l'extrémité distale du tube est équipée de moyens de connexion à un élément formant cathéter. Les moyens de connexion à un élément formant cathéter comportent avantageusement un embout de type luer-lock.

Le boîtier 3 comprend un manchon cylindrique axial 28 agencé pour recevoir par emboîtement à force l'extrémité proximale du tube 22, le tube étant évasé au niveau de son extrémité proximale de manière améliorer la fixation de ce dernier sur le boîtier.

Le tube 22 comporte un canal central dans lequel est engagé la broche creuse 16 reliée à l'orifice de sortie central 14 et des canaux parallèles et périphériques dans lesquels sont respectivement engagés les broches creuses 16 reliées aux orifices de sortie périphériques 15.

Ainsi, chaque conduit de passage 7 est destiné à être connecté respectivement à un récipient contenant du liquide et à un canal du tube 22.

Il doit également être noté que les conduits de passage 7 du boîtier 3 convergent vers le centre du boîtier dans une zone de diamètre correspondant sensiblement à celle du tube 22.

Le dispositif d'administration 2 comporte en outre une embase 31 sensiblement cylindrique sur laquelle est monté le boîtier 3.

Le boîtier 3 comporte une nervure annulaire 32 ménagée sur la face inférieure du flasque inférieur 5 et destinée à coopérer avec la surface intérieure de l'embase 31 de manière à permettre un assemblage du boîtier et de l'embase.

L'embase 31 comporte une douille axiale 33 de guidage du tube 22 reliée à la surface latérale interne de l'embase par une couronne circulaire 34 et des pattes de liaison 35.

L'embase 31 comporte également une ouverture radiale 32 agencée pour permettre le passage du tube 22. L'embase 31 comprend en outre sur sa surface extérieure des pattes d'accrochage 36 délimitant des logements pour le tube et permettant un enroulement du tube 22 sur deux tours autour de l'embase 31.

L'embase 31 forme ainsi un tambour d'enroulement pour le tube 22. Il doit être noté que la base du tambour d'enroulement est élargie afin de faciliter la préemption de celui-ci.

Il doit être noté que l'ouverture 32 est délimitée en partie par une portion de guidage courbée 37 agencée pour éviter un pincement du tube 22 contre la paroi latérale de l'embase 31 lors de son enroulement autour de celle-ci.

Selon un mode de réalisation de l'invention, le dispositif d'administration 2 pourrait comporter des capteurs montés sur la face extérieure du boîtier 3 dans des zones de réduction de matière 38, chaque capteur étant agencé pour mesurer une ou plusieurs valeurs physiques ou chimiques caractéristiques de l'écoulement de liquide dans un conduit de passage.

Selon un autre mode de réalisation de l'invention, chaque obturateur 17 pourrait est recouvert par un bouchon amovible vissé sur l'embout 9 correspondant.

Le procédé de montage et d'assemblage des différentes pièces constitutives du dispositif d'administration 2 va maintenant être décrit.

Ce procédé de montage et d'assemblage comporte les étapes suivantes consistant à :
- positionner les obturateurs 17, les embouts 9 et les pistons 12 dans les empreintes correspondantes 6 ménagées dans le flasque inférieur 5,
- monter le flasque supérieur 4 sur le flasque inférieur 5, le montage des deux flasques étant facilité par la présence de plots 41 et de logements 42 de positionnement ménagés respectivement sur les flasques supérieur 4 et inférieur 5,
- solidariser les flasques inférieur et supérieur, par exemple par soudage par ultrasons,
- insérer l'extrémité proximale du tube 22 dans l'ouverture radiale 32 ménagée dans l'embase 31 de l'extérieur vers l'intérieur, puis l'insérer dans la douille de retenue 33,
- connecter par emboîtement à force l'extrémité proximale du tube 22 dans le manchon 28 en engageant les broches 16 dans les canaux 30 du tube 22,
- monter le boîtier 3 sur l'embase 31 en faisant coopérer la nervure annulaire 32 avec la surface intérieure de l'embase,
- enrouler la portion de tube 22 faisant saillie de l'ouverture 32 autour de l'embase à l'aide des pattes d'accrochage 36.

Le fonctionnement du dispositif d'administration 2 va maintenant être décrit.

Tout d'abord, l'utilisateur raccorde l'extrémité distale du tube 22 à un élément formant cathéter positionné sur un patient à traiter, puis il connecte les différents récipients contenant les liquides de traitement médical à administer au patient aux embouts de type luer 9. Il doit être noté que chaque récipient comporte, au niveau de son extrémité connectée à l'embout 9 corrrespondant, une portion de distribution de liquide agencée pour pénétrer à travers la fente de la membrane 18 de l'obturateur correspondant 17.

Le liquide contenu dans chaque récipient s'écoule alors dans l'obturateur correspond 17, puis le long des rainures longitudinales 21 ménagée sur le piston correspondant 12, ensuite dans le canal de passage correspondant 13, dans le canal correspondant du tube 22 par l'intermédiaire de la broche correspondante 16, et enfin jusqu'à l'élément formant cathéter pour être enfin administré au patient.

Lorsque les différences de pression entre la pression veineuse et les pressions régnant dans les différents récipients provoquent un déplacement de liquide de la zone de convergence vers l'un des canaux du tube, le piston 12 correspondant est alors déplacé dans sa position d'obturation de l'extrémité ouverte de l'obturateur correspondant 17, ce qui empêche un passage de liquide de la zone de convergence vers le récipient correspondant, et donc évite un mélange de différents liquides dans ce récipient.

Il doit être noté que le retrait de chaque récipient connecté à un embout de type luer 9 provoque une dépression dans le conduits de passage 7 correspondant, en amont du piston 12 correspondant. Cette dépression engendre un déplacement du piston 12 dans sa position d'obturation de l'extrémité ouverte de l'obturateur correspondant 17, ce qui empêche une remontée de liquide de la zone de convergence vers le canal de passage 13 correspondant.

Ainsi, la présence d'un piston 12 dans chaque conduit de passage 7 permet d'empêcher, lors du retrait d'un récipient connecté au boîtier 3, une remontée de liquide de la zone de convergence vers le canal de passage 13 correspondant.

## Revendications

1. Dispositif d'administration (2) de plusieurs liquides de traitement médical à un patient humain ou animal, comportant un tube (22) contenant plusieurs canaux (30) sensiblement parallèles s'étendant sur toute la longueur du tube dont une extrémité, dite distale, est équipée de moyens de connexion à un élément formant cathéter, et dont l'autre extrémité, dite proximale, est connectée à un boîtier (3) équipé de moyens de connexion (9) de plusieurs récipients contenant chacun un liquide, le boîtier présentant des conduits de passage de liquide (7), chaque conduit de passage (7) étant destiné à être connecté respectivement à un récipient et à un canal du tube (22), le tube (22) comportant un canal central et des canaux parallèles et périphériques, **caractérisé en ce qu'**au moins un conduit de passage (7) du boîtier est équipe d'un clapet antiretour (12) agencé pour permettre le passage de liquide uniquement du récipient correspondant vers le canal correspondant du tube, et **en ce qu'**un des conduits (7) du boîtier comporte deux orifices de sortie (14, 15), respectivement en regard du canal central et d'un canal périphérique.

2. Dispositif d'administration selon la revendication 1, **caractérisé en ce que** les conduits de passage (7) du boîtier convergent vers le centre du boîtier dans une zone de diamètre correspondant sensiblement à celle du tube (22), chaque conduit (7) possédant un orifice de sortie (15) orienté perpendiculairement au plan dans lequel sont disposés les conduits, chaque orifice (15) étant en regard d'un canal du tube (22).

3. Dispositif d'administration selon la revendication 2, **caractérisé en ce que** chaque orifice de sortie (15) est prolongé par une broche creuse (16) engagée dans le canal du tube correspondant.

4. Dispositif d'administration selon l'une des revendications 1 à 3, **caractérisé en ce que** le boîtier (3) est de forme circulaire, et **en ce que** le tube (22) est connecté au boîtier de manière axiale.

5. Dispositif d'administration selon l'une des revendications 1 à 4, **caractérisé en ce que** le boîtier (3) comprend un manchon cylindrique (28) agencé pour recevoir par emboîtement à force l'extrémité proximale du tube (22).

6. Dispositif d'administration selon l'une des revendications 1 à 5, **caractérisé en ce que** chaque conduit (7) du boîtier est fermé au niveau de la paroi extérieure du boîtier par un obturateur (17) perforable, fendu ou à clapet mobile.

7. Dispositif d'administration selon la revendication 6, **caractérisé en ce que** chaque conduit (7) du boîtier délimite, au niveau de son extrémité tournée vers l'extérieur, un siège (8) dans lequel est monté un embout tubulaire (9) de type luer formant un moyen de connexion du récipient correspondant et servant au logement de l'obturateur correspondant (17).

8. Dispositif d'administration selon l'une des revendications 1 à 7, **caractérisé en ce que** chaque conduit (7) délimite une chambre (11) dans laquelle est monté mobile en translation un piston (12) formant clapet antiretour, le piston (12) étant mobile entre une première position permettant le passage de liquide du récipient correspondant vers le canal correspondant du tube et une seconde position empêchant le passage de liquide du canal correspondant du tube vers le récipient correspondant.

9. Dispositif d'administration selon les revendications 6 et 8, **caractérisé en ce que** chaque obturateur (17) est tubulaire et présente une première extrémité fermée et une seconde extrémité ouverte, et **en ce que** chaque piston (12) est agencé pour obturer l'extrémité ouverte de l'obturateur correspondant lorsqu'il est dans sa seconde position.

10. Dispositif d'administration selon la revendication 9, **caractérisé en ce que** chaque piston (12) présente une extrémité amincie (20) agencée pour obturer l'extrémité ouverte de l'obturateur correspondant (17) lorsque le piston (12) est dans sa seconde position.

11. Dispositif d'administration selon la revendication 10, **caractérisé en ce que** l'extrémité amincie (20) de chaque piston est tronconique.

12. Dispositif d'administration selon l'une des revendications 8 à 11, **caractérisé en ce que** chaque piston (12) présente un diamètre correspondant au diamètre de la chambre correspondante (11), et **en ce que** chaque piston comprend au moins une rainure longitudinale (21) ménagée sur sa surface extérieure et agencée pour permettre le passage de liquide du récipient correspondant vers le canal correspondant du tube.

13. Dispositif d'administration selon l'une des revendications 1 à 12, **caractérisé en ce qu'**il comporte une embase (31) sensiblement cylindrique sur laquelle est monté le boîtier (3), l'embase formant un tambour d'enroulement pour le tube (22).

## Patentansprüche

1. Verabreichungsvorrichtung (2) mehrerer medizinischer Behandlungsflüssigkeiten an einen Patienten, der ein Mensch oder Tier sein kann, die einen Schlauch (22) aufweist, der mehrere etwa parallele Kanäle (30) enthält, die sich über die gesamte Länge des Schlauchs erstrecken, von dem ein distales Ende mit Anschlussmitteln an ein Element ausgestattet ist, das einen Katheter bildet, und dessen anderes proximales Ende mit einem Gehäuse (3) verbunden ist, das mit Anschlussmitteln (9) mehrerer Behälter ausgestattet ist, die jeweils eine Flüssigkeit enthalten, wobei das Gehäuse Flüssigkeitsdurchgangsleitungen (7) aufweist, wobei jede Durchgangsleitung (7) dazu bestimmt ist, jeweils mit einem Behälter und mit einem Kanal des Schlauchs (22) verbunden zu sein, wobei der Schlauch (22) einen zentralen Kanal und parallele und periphere Kanäle aufweist, **dadurch gekennzeichnet, dass** mindestens eine Durchgangsleitung (7) des Gehäuses mit einer Rücklaufsperre (12) ausgestattet ist, die ausgebildet ist, um den Durchgang von Flüssigkeit nur vom entsprechenden Behälter zum entsprechenden Kanal des Schlauchs zu erlauben, und dass eine der Leitungen (7) des Gehäuses zwei Ausgangsöffnungen (14, 15) jeweils gegenüber dem zentralen Kanal und eines peripheren Kanals aufweist.

2. Verabreichungsvorrichtung nach Anspruch 1, **dadurch gekennzeichnet, dass** die Durchgangsleitungen (7) des Gehäuses zum Zentrum des Gehäuses in einer Zone mit einem Durchmesser konvergieren, die etwa der des Schlauchs (22) entspricht, wobei jede Leitung (7) eine Ausgangsöffnung (15) besitzt, die senkrecht zu der Ebene ausgerichtet ist, in der die Leitungen angeordnet sind, wobei jede Öffnung (15) gegenüber einem Kanal des Schlauchs (22) ist.

3. Verabreichungsvorrichtung nach Anspruch 2, **dadurch gekennzeichnet, dass** jede Ausgangsöffnung (15) von einem hohlen Stift (16) verlängert wird, der in den entsprechenden Kanal des Schlauchs eingeführt ist.

4. Verabreichungsvorrichtung nach einem der Ansprüche 1 bis 3, **dadurch gekennzeichnet, dass** das Gehäuse (3) rund ist und dass der Schlauch (22) mit dem Gehäuse axial verbunden ist.

5. Verabreichungsvorrichtung nach einem der Ansprüche 1 bis 4, **dadurch gekennzeichnet, dass** das Gehäuse (3) eine zylindrische Hülse (28) umfasst, die ausgebildet ist, um durch Einstecken kraftschlüssig das proximale Ende des Schlauchs (22) aufzunehmen.

6. Verabreichungsvorrichtung nach einem der Ansprüche 1 bis 5, **dadurch gekennzeichnet, dass** jede Leitung (7) des Gehäuses (7) auf Ebene der Außenwand des Gehäuses durch einen Verschluss (17) verschlossen ist, der perforierbar, geschlitzt oder mit beweglicher Klappe ist.

7. Verabreichungsvorrichtung nach Anspruch 6, **dadurch gekennzeichnet, dass** jede Leitung (7) des Gehäuses auf Ebene ihres Endes, das nach außen zeigt, einen Sitz (8) begrenzt, in den ein rohrförmiger Ansatz (9) vom Typ Luer montiert ist, der ein Anschlussmittel des entsprechenden Behälters bildet und für den entsprechenden Verschluss (17) als Aufnahme dient.

8. Verabreichungsvorrichtung nach einem der Ansprüche 1 bis 7, **dadurch gekennzeichnet, dass** jede Leitung (7) eine Kammer (11) begrenzt, in der ein Kolben(12), der die Rücklaufsperre bildet, in Verschiebung beweglich montiert ist, wobei der Kolben (12) zwischen einer ersten Stellung, die den Durchgang von Flüssigkeit aus dem entsprechenden Behälter in den entsprechenden Kanal des Schlauchs erlaubt, und einer zweiten Stellung, die den Durchgang von Flüssigkeit aus dem entsprechenden Kanal des Schlauchs in den entsprechenden Behälter verhindert, bewegbar ist.

9. Verabreichungsvorrichtung nach den Ansprüchen 6 und 8, **dadurch gekennzeichnet, dass** jeder Verschluss (17) rohrförmig ist und ein erstes geschlossenes Ende und ein zweites geöffnetes Ende aufweist, und dass jeder Kolben (12) ausgebildet ist, um das geöffnete Ende des entsprechenden Verschlusses zu verschließen, wenn er in seiner zweiten Stellung ist.

10. Verabreichungsvorrichtung nach Anspruch 9, **dadurch gekennzeichnet, dass** jeder Kolben (12) ein verschlanktes Ende (20) aufweist, das ausgebildet ist, um das geöffnete Ende des entsprechenden Verschlusses (17) zu verschließen, wenn der Kolben (12) in seiner zweiten Stellung ist.

11. Verabreichungsvorrichtung nach Anspruch 10, **dadurch gekennzeichnet, dass** das verschlankte Ende (20) jedes Kolbens kegelstumpfartig ist.

12. Verabreichungsvorrichtung nach einem der Ansprüche 8 bis 11, **dadurch gekennzeichnet, dass** jeder Kolben (12) einen Durchmesser aufweist, der dem Durchmesser der entsprechenden Kammer (11) entspricht, und dass jeder Kolben mindestens eine längliche Rille (21) umfasst, die auf seiner äußeren Fläche eingearbeitet ist und ausgebildet, um den Durchgang von Flüssigkeit aus dem entsprechenden Behälter in den entsprechenden Kanal des Schlauchs zu erlauben.

13. Verabreichungsvorrichtung nach einem der Ansprüche 1 bis 12, **dadurch gekennzeichnet, dass** sie eine etwa zylindrische Basis (31) aufweist, auf dem das Gehäuse (3) montiert ist, wobei die Basis eine Aufrolltrommel für den Schlauch (22) bildet.

## Claims

1. A device (2) for administering several medical treatment liquids to a human or animal patient, including a tube (22) containing several substantially parallel channels (30) extending over the entire length of the tube whereof one end (called the distal end) is equipped with means for connecting to a catheter-forming element, and the other end of which, called the proximal end, is connected to a housing (3) equipped with means (9) for connecting several containers each containing a liquid, the housing having liquid passage conduits (7), each passage conduit (7) being designed to be respectively connected to a container and a channel of the tube (22), the tube (22) including a central channel and parallel and peripheral channels, **characterized in that** at least one passage conduit (7) of the housing is equipped with a check valve (12) arranged to allow the passage of liquid only from the corresponding container for the corresponding channel of the tube, and **in that** one of the conduits (7) of the housing includes two output ports (14, 15), respectively across from the central channel and a peripheral channel.

2. The administration device according to claim 1, **characterized in that** the passage conduits (7) of the housing converge toward the center of the housing in a zone with a diameter substantially corresponding to that of the tube (22), each conduit (7) having an output port (15) oriented perpendicular to the plane in which the conduits are disposed, each port (15) being across from a channel of the tube (22).

3. The administration device according to claim 2, **characterized in that** each output port (15) is extended by a hollow pin (16) engaged in the channel of the corresponding tube.

4. The administration device according to one of claims 1 to 3, **characterized in that** the housing (3) is circular, and **in that** the tube (22) is connected axially to the housing.

5. The administration device according to one of claims 1 to 4, **characterized in that** the housing (3) comprises a cylindrical sleeve (28) arranged to receive the proximal end of the tube (22) by forcible fitting.

6. The administration device according to one of claims 1 to 5, **characterized in that** each conduit (7) of the housing is closed at the outer wall of the housing by a sealing member (17) that can be perforated, is slitted, or includes a moving valve.

7. The administration device according to claim 6, **characterized in that** each conduit (7) of the housing delimits, at the end thereof turned toward the outside, a seat (8) in which a tubular tip (9) of the Luer type is mounted forming means for connecting the corresponding container and serving as a housing for the corresponding sealing member (17).

8. The administration device according to one of claims 1 to 7, **characterized in that** each conduit (7) defines a chamber (11) in which a piston (12) is translatably mounted forming a check valve, the piston (12) being movable between a first position allowing the passage of liquid from the corresponding container to the corresponding channel of the tube, and a second position preventing the passage of liquid from the corresponding channel of the tube toward the corresponding container.

9. The administration device according to claims 6 and 8, **characterized in that** each sealing member (17) is tubular and has a first closed end and a second open end, and **in that** each piston (12) is arranged to seal the open end of the corresponding sealing member when it is in its second position.

10. The administration device according to claim 9, **characterized in that** each piston (12) has a thin end (20) arranged to seal the open end of the corresponding sealing member (17) when the piston (12) is in its second position.

11. The administration device according to claim 10, **characterized in that** the thin end (20) of each piston is tapered.

12. The administration device according to one of claims 8 to 11, **characterized in that** each piston (12) has a diameter corresponding to the diameter of the corresponding chamber (11), and **in that** each piston comprises at least one longitudinal slot (21 ) formed on the outer surface thereof and arranged to allow the passage of liquid from the corresponding container to the corresponding channel of the tube.

13. The administration device according to one of claims 1 to 12, **characterized in that** it includes a substantially cylindrical base (31) on which the housing (3) is mounted, the base forming a winding drum for the tube (22).
